# EUROPÄISCHE PATENTANMELDUNG

(11) **EP 4 706 729 A1**
(43) Veröffentlichungstag der Anmeldung: **11.03.2026**
(21) Anmeldenummer: 25200369.4
(22) Anmeldetag: 04.09.2025
(51) Int. Cl.: A61M 21/02, A61M 21/00

(54) **ELEKTRISCHE EINSCHLAFHILFE UND VERFAHREN ZU DEREN STEUERUNG, COMPUTERPROGRAMMPRODUKT SOWIE COMPUTER**

(30) Priorität: 04.09.2024 DE 102024125356
(71) Anmelder: Loume Tech GmbH, 20354 Hamburg (DE)
(72) Erfinder: Schnauer, Markus Harm, 20354 Hamburg (DE); Dik, Viktor, 20354 Hamburg (DE)
(74) Vertreter: Launhardt, Thomas

(57) **Zusammenfassung**

Die vorliegende Offenbarung betrifft eine elektrische Einschlafhilfe (1; 1'). Die elektrische Einschlafhilfe (1; 1') hat eine Lichtquelle (2), eine Tonquelle (3) und eine Steuereinrichtung (4; 4'). Die Lichtquelle (2) ist eingerichtet, ein sichtbares Licht zu erzeugen und die Helligkeit des Lichts zu ändern. Die Tonquelle (3) ist eingerichtet, hörbare Töne und/oder hörbare Geräusche zu erzeugen. Die Steuereinrichtung (4; 4') ist eingerichtet, nach einer vorgegebenen Einschlafroutine sowohl die Lichtquelle (1) zum Ändern der Helligkeit des Lichts anzusteuern, um ein Einatmen oder Ausatmen einer geführten Atemmeditation visuell darzustellen, als auch die Tonquelle (3) zum Erzeugen einer vorzugsweise gesprochenen und/oder musikalischen Tonfolge und/oder wenigstens eines Geräusches anzusteuern, um eine geführte Audiomeditation akustisch darzustellen. Die vorliegende Offenbarung betrifft ferner ein Verfahren zur Steuerung der elektrischen Einschlafhilfe (1; 1'), ein Computerprogrammprodukt und einen Computer, insbesondere ein Smartphone (20).

## Beschreibung

Die vorliegende Offenbarung betrifft eine elektrische Einschlafhilfe und ein Verfahren zu deren Steuerung. Die vorliegende Offenbarung betrifft ferner ein Computerprogrammprodukt sowie einen Computer, wie beispielsweise ein Smartphone.

Ein gesunder Schlaf ist unabdingbar für Wachstum, Regeneration und das Gedächtnis. Im Schlaf erholt sich der Körper, die Muskeln entspannen und die Erlebnisse und Impulse des Tages werden verarbeitet und gespeichert. Der Schlaf als Ganzes, vom Zubettgehen bis zum Aufstehen, kann in vier Phasen unterteilt werden: die Vorbereitung, das Einschlafen, der Schlaf und das Aufwachen.

Viele Menschen leiden darunter, dass sie gar nicht oder nur schwer einschlafen können. Nicht zuletzt dadurch fühlen sie sich tagsüber oft müde und weniger leistungsfähig. Auch vor diesem Hintergrund besteht ein großes Interesse, ein Konzept zu entwickeln, welches das Einschlafen erleichtert.

Diesbezüglich wird eine elektrische Einschlafhilfe vorgeschlagen. Die elektrische Einschlafhilfe umfasst eine Lichtquelle, eine Tonquelle und eine Steuereinrichtung. Die Lichtquelle ist insbesondere eingerichtet, ein sichtbares Licht zu erzeugen und die Helligkeit des Lichts zu ändern. Die Tonquelle ist insbesondere eingerichtet, hörbare Töne und/oder hörbare Geräusche zu erzeugen. Die Steuereinrichtung ist insbesondere eingerichtet, nach wenigstens einer vorzugsweise vorgegebenen Einschlafroutine, insbesondere ausschließlich nach einer vorzugsweise vorgegebenen Einschlafroutine, sowohl die Lichtquelle zum Ändern der Helligkeit des Lichts anzusteuern, beispielsweise um ein Einatmen oder Ausatmen einer geführten Atemmeditation visuell darzustellen, als auch die Tonquelle zum Erzeugen wenigstens einer vorzugsweise gesprochenen und/oder musikalischen Tonfolge und/oder wenigstens eines Geräusches anzusteuern, beispielsweise um eine geführte Audiomeditation akustisch darzustellen.

Durch die vorgeschlagene Einschlafhilfe sind anhand von visuellen und akustischen Signalen zwei psychotherapeutische Ansätze ermöglicht, um eine Person zum Einschlafen zu bringen oder zumindest beim Einschlafen zu unterstützen. Es können geführte Atemübungen anhand visueller Signale und/oder akustischer Signale ausgeführt werden. Ergänzend oder alternativ kann eine geführte Audiomeditation anhand akustischer Signale ausgeführt werden. Durch die vorgeschlagene Einschlafhilfe können diese beiden Ansätze mittels der Steuereinrichtung und der vorgegebenen Einschlafroutine, je nach Bedarf und Erfordernis, in Kombination oder einzeln, beispielsweise zeitliche getrennt voneinander oder zeitlich gemeinsam, umgesetzt werden.

Unter dem Begriff "sichtbares Licht" ist in der vorliegenden Offenbarung ein Wellenlängenbereich des elektromagnetischen Spektrums zu verstehen, der beim Menschen Hell- und/oder Farbempfindungen hervorruft. Beispielsweise hat das sichtbare Licht eine Wellenlänge zwischen 360 Nanometer und 830 Nanometer, insbesondere zwischen 400 Nanometer und 750 Nanometer.

Unter dem Begriff "hörbare Töne" sind in der vorliegenden Offenbarung vom Gehör wahrgenommene Schallereignisse zu verstehen, welche eine regelmäßige bzw. periodische Schwingung aufweisen. Beispielsweise fallen darunter solche Schallereignisse, welche durch Lebewesen und/oder Musikinstrumente und/oder andere Objekte erzeugt werden. Beispielsweise liegen die hörbaren Geräusche in einer Frequenz zwischen 20 Hertz und 20 Kilohertz vor.

Unter dem Begriff "hörbare Geräusche" sind in der vorliegenden Offenbarung vom Gehör wahrgenommene Schallereignisse zu verstehen, welche unregelmäßige Schwingungen vorzugsweise ohne bestimmte Tonhöhe aufweisen. Beispielsweise ändern sich die Frequenz und die Lautstärke über die Zeit und unterliegen keiner Gesetzmäßigkeit. Beispielsweise liegen die hörbaren Geräusche in einer Frequenz zwischen 20 Hertz und 20 Kilohertz vor.

Unter dem Begriff "Tonfolge" ist in der vorliegenden Offenbarung insbesondere eine Aneinanderreihung von Tönen zu verstehen. Unter dem Begriff "klangliche Tonfolge" ist in der vorliegenden Offenbarung insbesondere eine Tonfolge zu verstehen, bei der die aneinandergereihten Töne in ihrer Klangfarbe und/oder ihren klanglichen Eigenschaften zueinander in einer Beziehung stehen.

Unter dem Begriff "gesprochene Tonfolge" ist in der vorliegenden Offenbarung insbesondere eine Tonfolge, beispielsweise ein Tonverlauf oder eine Sprachmelodie, in einer gesprochenen Sprache zu verstehen. Unter dem Begriff "musikalische Tonfolge" ist in der vorliegenden Offenbarung insbesondere eine Tonfolge zu verstehen, durch welche eine Melodie oder ein Motiv oder eine Tonleiter erzeugt wird.

Unter dem Begriff "geführte Meditation" ist in der vorliegenden Offenbarung zu verstehen, dass die Aufmerksamkeit einer Person durch einen Dritten, wie beispielsweise die vorgeschlagene Einschlafhilfe, trainiert wird und zwar durch visuelle und/oder akustische Signale geleitet, also geführt wird.

Unter dem Begriff "geführte Audiomeditation" ist in der vorliegenden Offenbarung zu verstehen, dass die geführte Meditation durch akustische, insbesondere hörbare Signale, wie beispielsweise eine vorzugsweise klangliche Tonfolge, insbesondere eine gesprochene und/oder musikalische Tonfolge, und/oder wenigstens ein Geräusch bewirkt wird.

Unter dem Begriff "geführte Atemmeditation" ist in der vorliegenden Offenbarung zu verstehen, dass die geführte Meditation durch visuelle Signale und/oder durch akustische, insbesondere hörbare Signale, wie beispielsweise eine vorzugsweise klangliche Tonfolge, insbesondere eine gesprochene und/oder musikalische Tonfolge, und/oder wenigstens ein Geräusch bewirkt wird und der Fokus der Meditation auf der Atmung liegt. Auch eine geführte Audiomeditation kann eine geführte Atemmeditation sein oder umfassen.

Unter dem Begriff "geführte Atemübung" ist in der vorliegenden Offenbarung zu verstehen, dass die Atmung durch einen Dritten, wie beispielsweise die vorgeschlagene Einschlafhilfe, trainiert wird und zwar durch visuelle und/oder akustische Signale geleitet, also geführt wird. Die geführte Atemübung kann durch eine geführte Atemmeditation bewirkt sein. Auch kann eine geführte Atemübung durch eine geführte Audiomeditation bewirkt sein.

Unter dem Begriff "Einschlafroutine" ist in der vorliegenden Offenbarung insbesondere eine Anleitung zum Ansteuern der Lichtquelle und/oder der Tonquelle zu verstehen, beispielsweise um die geführte Meditation visuell und/oder akustisch darzustellen, insbesondere den Vorgang bzw. Ablauf der geführten Meditation visuell und/oder akustisch darzustellen.

Die Einschlafroutine kann eine Anleitung zum Ansteuern der Lichtquelle und/oder der Tonquelle sein oder umfassen, beispielsweise um eine Atemübung visuell und/oder akustisch darzustellen. Die Atemübung kann die sogenannte 4-7-8-Atemübung sein, bei der 4 Sekunden einzuatmen, anschließend 7 Sekunden die Luft anzuhalten und dann 8 Sekunden auszuatmen ist. Die Anleitung kann eine vorgegebene Anzahl an Wiederholungen der Atemübung enthalten.

Die Einschlafroutine ist bevorzugt vorgebbar oder vorgegeben. Grundsätzlich können auch mehrere Einschlafroutinen bereitgestellt sein. Beispielsweise sind die mehreren Einschlafroutinen zueinander unterschiedlich. Beispielsweis hat der Nutzer der Einschlafhilfe die Möglichkeit, eine der bereitgestellten Einschlafroutinen auszuwählen.

Die Steuereinrichtung ist insbesondere eingerichtet, ausschließlich nach der wenigstens einen Einschlafroutine die Lichtquelle und die Tonquelle anzusteuern. Unter dem Begriff "ausschließlich" ist in diesem Zusammenhang in der vorliegenden Offenbarung insbesondere zu verstehen, dass die Steuereinrichtung nur nach der wenigstens einen Einschlafroutine die Lichtquelle und/oder die Tonquelle ansteuert, ohne dass der momentane Zustand des Nutzers der Einschlafhilfe berücksichtigt wird.

Unter dem Begriff "Steuereinrichtung" ist in der vorliegenden Offenbarung insbesondere eine elektrische Steuereinrichtung zu verstehen. Beispielsweise ist die Steuereinrichtung eine elektronische Einrichtung einer Computer-Hardware, welche in Bezug auf die Lichtquelle und die Tonquelle bestimmte Vorgänge und/oder Abläufe steuert. Die Steuereinrichtung kann eine digitale Verarbeitungseinheit aufweisen, die beispielsweise eine Mikroprozessoreinheit (CPU) umfasst. Die CPU kann mit einem Speichersystem und/oder Bussystem daten- und/oder signalverbunden sein. Die Steuereinrichtung kann ein oder mehrere Programme oder Programmmodule aufweisen.

Die digitale Verarbeitungseinheit kann derart ausgebildet sein, dass Befehle, die als ein in einem Speichersystem abgelegtes Programm implementiert sind, abgearbeitet, Eingangssignale von einem Datenbussystem entgegengenommen und/oder Ausgangssignale an ein Datenbussystem abgegeben werden. Ein Speichersystem kann ein oder mehrere, insbesondere verschiedene, Speichermedien aufweisen. Die Speichermedien können insbesondere optische, magnetische, Festkörper-Speichermedien und/oder andere, vorzugsweise nichtflüchtige Speichermedien sein.

Bei einer Ausführungsform ist die Steuereinrichtung eingerichtet, die Lichtquelle zum Ändern der Helligkeit des Lichts ins Hellere anzusteuern, beispielsweise um eine Atemrichtung visuell darzustellen. Ergänzend oder alternativ kann die Steuereinrichtung eingerichtet sein, die Lichtquelle zum Ändern der Helligkeit des Lichts ins Dunklere anzusteuern, beispielsweise um eine andere Atemrichtung visuell darzustellen. Dadurch ist eine bewusste Wahrnehmung des visuellen Signals der Lichtquelle in Bezug auf die Atemrichtung begünstigt. Das kann sowohl das visuelle Signal für das Einatmen als auch das visuelle Signal für das Ausatmen betreffen.

Beispielsweise ist die Steuereinrichtung eingerichtet, die Lichtquelle zum Ändern der Helligkeit des Lichts ins Hellere anzusteuern, um ein Einatmen visuell darzustellen. Beispielsweise ist die Steuereinrichtung eingerichtet, die Lichtquelle zum Ändern der Helligkeit des Lichts ins Dunklere anzusteuern, um ein Ausatmen visuell darzustellen. Grundsätzlich kann die Steuereinrichtung auch eingerichtet sein, die Lichtquelle zum Ändern der Helligkeit des Lichts ins Dunklere anzusteuern, um ein Einatmen visuell darzustellen bzw. die Lichtquelle zum Ändern der Helligkeit des Lichts ins Hellere anzusteuern, um ein Ausatmen visuell darzustellen.

Die Steuereinrichtung kann eingerichtet sein, die Lichtquelle derart anzusteuern, dass die Helligkeit des Lichts über eine vorgegebene Zeitdauer sich ändert, insbesondere heller oder dunkler wird, um einen Atemvorgang visuell darzustellen. Dadurch ist eine geführte Atemmeditation begünstigt, da die Dauer eines Atemvorgangs zeitlich vorgegeben und, wenn erwünscht, auch zeitlich verändert werden kann.

Die Steuereinrichtung kann ferner eingerichtet sein, die Lichtquelle derart anzusteuern, dass die Geschwindigkeit der Änderung der Helligkeit des Lichts über den vorgegebenen Zeitraum sich ändert, insbesondere zunimmt oder abnimmt. Auch dadurch ist eine geführte Atemmeditation begünstigt. Denn durch eine schnellere oder langsamere Änderung der Helligkeit des Lichts, kann der Atemvorgang in einer gewünschten Art und Weise gezielt beeinflusst werden.

Ergänzend oder alternativ kann die Steuereinrichtung eingerichtet sein, die Lichtquelle derart anzusteuern, dass die Helligkeit des Lichts über eine vorgegebene Zeitdauer gleichbleibend ist, um einen Atemvorgang visuell darzustellen. Dadurch ist eine bewusste Wahrnehmung des visuellen Signals der Lichtquelle dahingehend begünstigt, dass ein Atemvorgang als solches sich auf diese Weise abbilden lässt.

Weiter kann die Steuereinrichtung eingerichtet sein, die Tonquelle zeitabhängig anzusteuern. Dadurch ist eine geführte Audiomeditation begünstigt, da die dafür erzeugte Tonfolge zeitlich vorgegeben und, wenn erwünscht, auch zeitlich verändert werden kann. Bevorzugt ist die Steuereinrichtung eingerichtet, die Lichtquelle und die Tonquelle zeitabhängig anzusteuern.

Beispielsweise ist die Steuereinrichtung eingerichtet, in Abhängigkeit wenigstens eines vorgegebenen Zeitpunktes die Lichtquelle und/oder die Tonquelle anzusteuern, insbesondere wiederkehrend anzusteuern, beispielsweise um eine Information über ein bevorstehendes Einschlafen visuell und/oder akustisch auszugeben. Dadurch ist es ermöglicht, den Nutzer an eine bevorstehende Schlafenszeit zu erinnern. Beispielsweise kann auf diese Art und Weise der Nutzer täglich durch visuelle und/oder akustische Signale an eine vorher vorgegebene Schlafenszeit erinnert werden.

Es bietet sich an, dass die Einschlafroutine eine Datenbasis mit Informationen über Zeitpunkte zum Ansteuern der Lichtquelle und Informationen über Zeitpunkte zum Ansteuern der Tonquelle hat. Beispielsweise ist in diesem Fall die Steuereinrichtung eingerichtet, nach dieser Datenbasis, also nach diesen Informationen, die Lichtquelle und die Tonquelle anzusteuern, insbesondere gemeinsam anzusteuern. Dazu können die Zeitpunkte zum Ansteuern der Lichtquelle und die Zeitpunkte zum Ansteuern der Tonquelle aufeinander abgestimmt sein, beispielsweise um ein Einatmen oder Ausatmen visuell darzustellen, wenn eine zugehörige vorzugsweise gesprochene und/oder musikalische Tonfolge und/oder wenigstens ein zugehöriges Geräusch erfolgt ist oder erfolgt.

Bei einer weiteren Ausführungsform umfasst die vorgeschlagene Einschlafhilfe eine Projektionseinrichtung. Die Projektionseinrichtung ist eingerichtet, eine geometrische Figur auf eine Fläche, insbesondere eine Wandfläche oder eine Deckenfläche beispielsweise eines Schlafraumes, zu projizieren. Dazu nutzt die Projektionseinrichtung die Lichtquelle als Beleuchtungsquelle zum Beleuchten einer Projektionsvorlage. Auf diese Weise ist es für einen Nutzer erleichtert, das Licht der Einschlafhilfe zu verfolgen. Dies ist beispielsweise der Fall, wenn die geometrische Figur an die Decke projiziert wird. Der Benutzer kann dann, beispielsweise von seinem Bett aus, die geometrische Figur verfolgen, ohne dass er dazu seine Einschlafposition verlassen braucht. Beispielsweise liegt der Benutzer rückenliegend in seinem Bett und schaut nach oben gegen die Decke, gegen welche die geometrische Figur projiziert ist.

Die vorgeschlagene Einschlafhilfe kann so beschaffen sein, dass die Projektionsvorlage eine Blendenöffnung und ein darin vorzugsweise konzentrisch angeordnetes Innenteil umfasst, welches kleiner als die Blendenöffnung ausgebildet und lichtundurchlässig ist. Dadurch weist die projizierte geometrische Figur eine Ringkontur auf. Es hat sich herausgestellt, dass die Wahrnehmung der projizierten Figur begünstig ist, wenn die projizierte Figur eine Ringkontur hat. Besondere Aufmerksamkeit beim Nutzer zeigte sich, wenn die projizierte Figur die Kontur eines runden Ringes, insbesondere Kreisrings hatte. Es bietet sich daher an, dass die Blendenöffnung und das Innenteil rund, beispielsweise kreisrund sind.

Auch bietet es sich an, dass die Blendenöffnung und das Innenteil oval sind. Es hat sich gezeigt, erst mit einer solchen geometrischen Form etwaige entstehenden Verzerrungen soweit ausgeglichen werden können, dass die projizierte geometrische Figur in Form eines Kreisrings in Erscheinung tritt.

Bei einer weiteren Ausführungsform umfasst die vorgeschlagene Einschlafhilfe eine Hintergrundbeleuchtung. Die Hintergrundbeleuchtung dient insbesondere dazu, einen um die geometrische Figur herum verlaufenden Fläche zu beleuchten. Es hat sich gezeigt, dass sich dadurch bei dem Nutzer der Einschlafhilfe der Grad der Entspannung weiter erhöht. Insofern fördert bzw. unterstützt auch diese Maßnahme das Einschlafen. Die Hintergrundbeleuchtung kann eine Ambientebeleuchtung sein. Beispielsweise umfasst die Hintergrundbeleuchtung eine weitere Lichtquelle und eine weitere Blendenöffnung. Beispielsweise ist die weitere Blendenöffnung koaxial zur Blendenöffnung der Projektionsvorlage angeordnet und größer als die Blendenöffnung der Projektionsvorlage ausgebildet.

Bei einer weiteren Ausführungsform umfasst die vorgeschlagene Einschlafhilfe ein, beispielsweise einteiliges oder mehrteiliges Gerätegehäuse, in dem die Lichtquelle und die Tonquelle aufgenommen sind. Insbesondere ist auch die Projektionseinrichtung in dem Gerätegehäuse aufgenommen. Insbesondere umfasst das Gerätegehäuse eine Aufstandsfläche zum Aufstellen auf einem Untergrund, wie beispielsweise einem Nachttisch. Beispielsweise ist die Aufstandsfläche gegenüberliegend oder im Wesentlichen gegenüberliegend zu der Blendenöffnung der Projektionseinrichtung angeordnet. Dadurch ist bei aufgestelltem Gerät auf dem Untergrund eine Projektion an die Decke eines Raumes oder einen deckennahen Wandbereich ermöglicht.

Die vorgeschlagene Einschlafhilfe kann so beschaffen sein, dass die Steuereinrichtung dem Gerätegehäuse zugeordnet ist, insbesondere darin aufgenommen ist. Für diesen Fall bietet es sich an, dass die Steuereinrichtung mit der Lichtquelle und der Tonquelle kabelgebunden signalverbunden ist oder eingerichtet ist, mit der Lichtquelle und der Tonquelle kabelgebunden signalverbunden zu werden.

Alternativ kann die vorgeschlagene Einschlafhilfe auch so beschaffen sein, dass die Steuereinrichtung ein Bestandteil eines vorzugsweise separaten Endgerätes und/oder eines vorzugsweise separaten Computers ist. Beispielsweise ist die Steuereinrichtung ein Bestandteil eines Smartphones, Tablets oder Notebooks. Bei dieser Alternative bietet es sich an, dass die Steuereinrichtung eingerichtet ist, mit der Lichtquelle und/oder der Tonquelle vorzugsweise kabellos signalverbunden zu werden.

Die vorgeschlagene Einschlafhilfe kann so beschaffen sein, dass die Lichtquelle wenigstens eine LED umfasst. Die wenigstens eine LED kann eine RGB-LED sein. Eine solche LED verwendet beispielsweise drei Leuchtdioden in den Farben Rot, Grün und Blau und kann diese in unterschiedlichen Intensitäten zu verschiedenfarbigem Licht vermischen.

Die vorgeschlagene Einschlafhilfe kann ferner so beschaffen sein, dass die Tonquelle wenigstens einen Lautsprecher umfasst. Der wenigstens eine Lautsprecher ist beispielsweise ein Membranlautsprecher.

Nach einem Aspekt wird ein Verfahren zur Steuerung der Steuereinrichtung der vorstehend beschriebenen Einschlafhilfe vorgeschlagen. Durch das Verfahren können die vorstehend zur Einschlafhilfe beschriebenen Vorteile erreicht werden.

Bei einer Ausführungsform des Verfahrens wird mittels der Steuereinrichtung nach einer vorgegebenen Einschlafroutine sowohl die Lichtquelle zum Ändern der Helligkeit des Lichts angesteuert, um ein Einatmen oder Ausatmen einer geführten Atemmeditation visuell darzustellen, als auch die Tonquelle zum Erzeugen einer vorzugsweise gesprochenen und/oder musikalischen Tonfolge und/oder wenigstens eines Geräusches angesteuert, um eine geführte Audiomeditation akustisch darzustellen. Die vorgegebene Einschlafroutine kann die vorstehend beschriebene Einschlafroutine sein.

Nach einem weiteren Aspekt wird ein Computerprogrammprodukt mit einem Programmcode vorgeschlagen, insbesondere zur Durchführung des vorstehend beschriebenen Verfahrens zur Steuerung der Steuereinrichtung der vorgeschlagenen Einschlafhilfe. Beispielsweise ist der Programmcode auf einem von einem Computer lesbaren Medium gespeichert ist. Beispielsweise weist der Programmcode einen Computer oder den vorgenannten Computer zur Durchführung der Schritte des vorstehend beschriebenen Verfahrens zur Steuerung der Steuereinrichtung der vorgeschlagenen Einschlafhilfe an, wenn der Programmcode auf dem Computer ausgeführt wird.

Nach einem weiteren Aspekt wird ein Computer vorgeschlagen, welcher die vorstehend beschriebene Steuereinrichtung umfasst und das vorstehend beschriebene Computerprogrammprodukt hat. Beispielsweise ist der Computer ein Smartphone oder eine Hardwarekomponente eines Smartphones.

Weitere Einzelheiten und Merkmale ergeben sich aus der nachfolgenden Beschreibung mehrerer Ausführungsbeispiele anhand der Zeichnung. Es zeigen
- Fig. 1: eine erste exemplarische Ausführungsform einer elektrischen Einschlafhilfe in einer perspektivischen Ansicht,
- Fig. 2: die exemplarische Einschlafhilfe der Figur 1 in einer Schnittdarstellung,
- Fig. 3: einen Ausschnitt aus einem Deckelteil der exemplarischen Einschlafhilfe der Figur 1,
- Fig. 4: beispielhaft ein mit der exemplarischen Einschlafhilfe der Figur 1 beleuchteter Raum,
- Fig. 5: einen Ausschnitt einer von der exemplarischen Einschlafhilfe der Figur 1 beleuchtete Fläche,
- Fig. 6: beispielhaft eine geführte Atemübung, dargestellt durch ein visuelles Signal einer Lichtquelle der exemplarischen Einschlafhilfe gemäß der Figur 1, und
- Fig. 7: eine zweite exemplarische Ausführungsform einer elektrischen Einschlafhilfe in einer perspektivischen Ansicht.

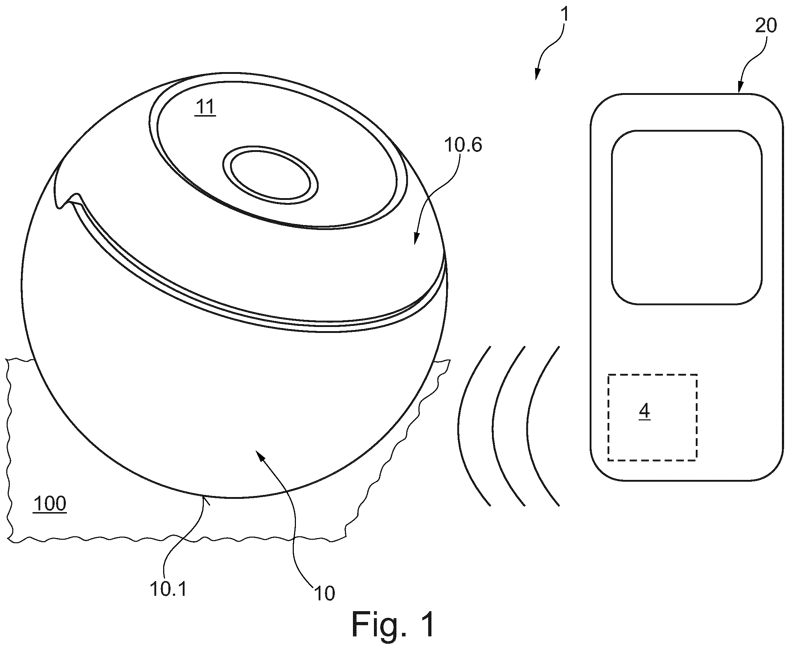

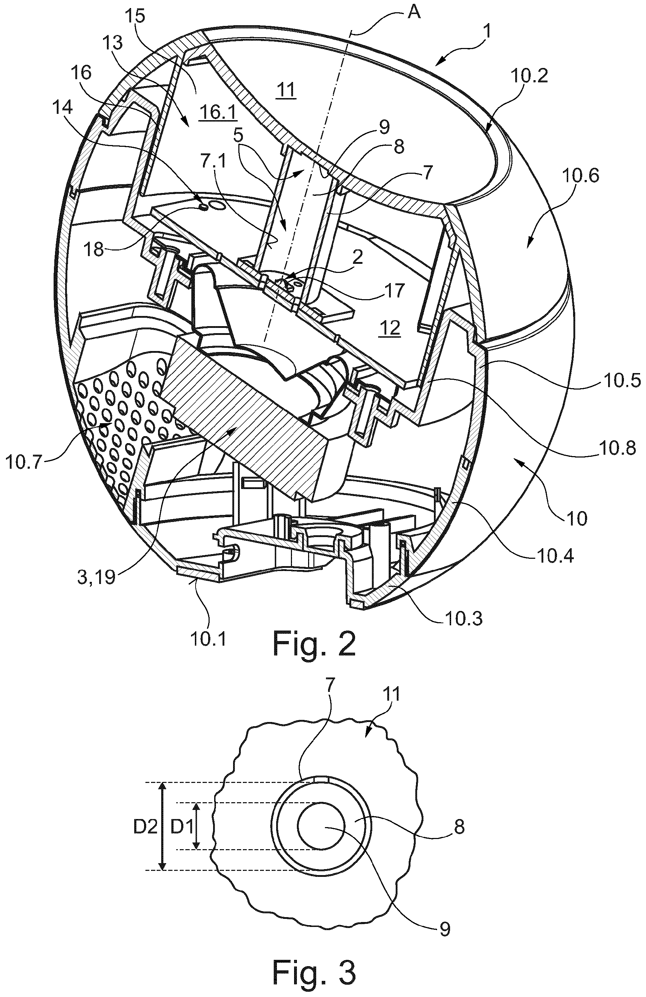

Figuren 1 und 2 zeigen - in schematischer Darstellung - eine erste exemplarische Ausführungsform einer elektrischen Einschlafhilfe 1 in einer perspektivischen Ansicht (Figur 1) und in einem Querschnitt (Figur 2). Die elektrische Einschlafhilfe 1 dient dazu, das Einschlafen einer Person zu unterstützen bzw. zu fördern.

Die exemplarische Einschlafhilfe 1 umfasst eine Lichtquelle 2, insbesondere eine elektrische Lichtquelle. Bevorzugt ist die Lichtquelle 2 eingerichtet, ein sichtbares Licht, insbesondere ein Weißlicht, zu erzeugen und vorzugsweise die Helligkeit des Lichts ändern zu können. Die exemplarische Einschlafhilfe 1 umfasst ferner eine Tonquelle 3, insbesondere eine elektrische Tonquelle. Bevorzugt ist die Tonquelle 3 eingerichtet, hörbare Töne und/oder hörbare Geräusche zu erzeugen.

Weiter umfasst die exemplarische Einschlafhilfe 1 eine Steuereinrichtung, insbesondere elektrische Steuereinrichtung 4. Bevorzugt ist die Steuereinrichtung 4 eingerichtet, die Lichtquelle 2 zum Ändern der Helligkeit des Lichts anzusteuern, um ein Einatmen oder Ausatmen einer geführten Atemmeditation visuell darzustellen.

Bevorzugt ist die Steuereinrichtung 4 ferner eingerichtet, die Tonquelle 3 zum Erzeugen wenigstens einer Tonfolge und/oder wenigstens eines Geräusches anzusteuern, um eine geführte Audiomeditation akustisch darzustellen. Die wenigstens eine Tonfolge kann eine gesprochene Tonfolge sein oder enthalten. Die wenigstens eine Tonfolge kann auch eine musikalische Tonfolge sein oder enthalten. Grundsätzlich kann die wenigstens eine Tonfolge auch eine Kombination einer gesprochenen Tonfolge und einer musikalischen Tonfolge sein oder enthalten.

Bevorzugt ist die Steuereinrichtung 4 eingerichtet, die Lichtquelle 2 zum Ändern der Helligkeit des Lichts ins Hellere anzusteuern, um eine Atemrichtung visuell darzustellen. Bevorzugt ist die Steuereinrichtung 4 ferner eingerichtet, die Lichtquelle 2 zum Ändern der Helligkeit des Lichts ins Dunklere anzusteuern, um eine andere Atemrichtung visuell darzustellen.

Bevorzugt ist die Steuereinrichtung 4 eingerichtet, die Lichtquelle 2 und/oder die Tonquelle 3 zeitabhängig anzusteuern. Bevorzugt ist die Steuereinrichtung 4 eingerichtet, die Lichtquelle derart anzusteuern, dass die Helligkeit des Lichts über wenigstens eine vorgegebene Zeitdauer sich ändert, beispielsweise heller oder dunkler wird, um einen Atemvorgang visuell darzustellen. Bevorzugt ist die Steuereinrichtung 4 ferner eingerichtet, die Lichtquelle 2 derart anzusteuern, dass die Geschwindigkeit der Änderung der Helligkeit des Lichts über die wenigstens eine vorgegebene Zeitdauer sich ändert, beispielsweise zunimmt oder abnimmt.

Die Steuereinrichtung 4 kann auch eingerichtet sein, in Abhängigkeit wenigstens eines vorgegebenen Zeitpunktes die Lichtquelle 2 und/oder die Tonquelle 3 anzusteuern, insbesondere wiederkehrend anzusteuern, um eine Information über ein bevorstehendes Einschlafen visuell und/oder akustisch auszugeben. Dies ermöglicht, den Nutzer an eine bevorstehende Schlafenszeit zu erinnern, insbesondere regelmäßig zu erinnern.

Bevorzugt nutzt die Steuereinrichtung 4 zum Ansteuern der Lichtquelle 2 und/oder der Tonquelle 3 eine vorgegebene Einschlafroutine, steuert also die Lichtquelle 2 und/oder die Tonquelle 3 nach der vorgegebenen Einschlafroutine an. Bevorzugt hat die vorgegebene Einschlafroutine eine Datenbasis mit Informationen über Zeitpunkte zum Ansteuern der Lichtquelle 2 und Informationen über Zeitpunkte zum Ansteuern der Tonquelle 3. Bevorzugt sind die Zeitpunkte zum Ansteuern der Lichtquelle 2 und die Zeitpunkte zum Ansteuern der Tonquelle 3 aufeinander abgestimmt, beispielsweise in der Art und Weise, dass ein Einatmen oder Ausatmen visuell dargestellt wird, wenn ein zugehöriges Tonsignal, also eine gesprochene und/oder musikalische Tonfolge, und/oder wenigstens ein zugehöriges Geräusch erfolgt ist bzw. sind oder erfolgt.

Die vorgegebene Einschlafroutine kann ein Bestandteil einer Software sein, welche mit der Steuereinrichtung 4 zusammenwirkt, beispielsweise auf dem Computer, insbesondere dem Smartphone 20, implementiert ist. Beispielsweise ist die Software eine herunterladbare App oder Bestandteil einer solchen Software.

Die exemplarische Einschlafhilfe 1 ermöglicht anhand der Lichtquelle 2, der Tonquelle 3 und der Steuereinrichtung 4, insbesondere der Funktionen der Steuereinrichtung 4, eine Kombination von zwei psychotherapeutischen Ansätzen, um eine Person zum Einschlafen zu bringen oder zumindest beim Einschlafen zu unterstützen. Zur Unterstützung des Einschlafens bietet die exemplarische Einschlafhilfe 1 eine geführte Atemmeditation, welche visuell durch Lichtsignale der Lichtquelle 2 und/oder akustisch durch Tonsignale bzw. Geräuschsignale der Tonquelle 3 realisiert werden kann.

Beispielsweise kann auf diese Weise eine 4-7-8 Atemtechnik dargestellt werden, bei der der Nutzer dahin gehend geführt wird, dass vier Sekunden eingeatmet, anschließend sieben Sekunden die Luft angehalten und danach acht Sekunden ausgeatmet werden soll. Zur Unterstützung des Einschlafens bietet die exemplarische Einschlafhilfe auch die Möglichkeit einer geführten Audiomeditation durch gesprochene und/oder musikalische Tonsignale und/oder durch Geräusche. Die geführte Audiomeditation ermöglicht, die Aufmerksamkeit der Person auf das Einschlafen zu richten und dabei auch andere Aspekte als die Atmung einzubeziehen.

Bei der exemplarischen Einschlafhilfe 1 sind die Lichtquelle 2 und die Tonquelle 3 einem Gerätegehäuse 10 zugeordnet, insbesondere gemeinsam zugeordnet. Bevorzugt ist die Lichtquelle 2 in dem Gerätegehäuse 10, insbesondere innerhalb des Gerätegehäuses 10 angeordnet. Bevorzugt ist die Tonquelle 3 in dem Gerätegehäuse 10, insbesondere innerhalb des Gerätegehäuses 10 angeordnet. Die Steuereinrichtung 4 kann außerhalb des Gerätegehäuses 10 vorliegen.

Bei der exemplarischen Einschlafhilfe 1 ist die Steuereinrichtung 4 beispielsweise einem Smartphone 20 zugeordnet, insbesondere ein integraler Bestandteil des Smartphones 20. Grundsätzlich kann die Steuereinrichtung 4 jedem beliebigen Computer zugeordnet sein bzw. durch jeden beliebigen Computer bereitgestellt sein, wobei der Computer stationär oder mobil sein kann, also auch ein Notebook oder Tablet sein kann.

Bevorzugt weist das Gerätegehäuse 10 eine vorzugsweise ebene Aufstandsfläche 10.1 zum Aufstellen auf einem Untergrund 100 auf. Der Untergrund 100 kann eine Aufstellfläche eines Nachttischs oder sonstigen Möbelstücks sein. Auch kann der Untergrund 100 eine Bodenfläche des Raumes sein. Durch die Aufstandsfläche 10.1 ist ein sicherer Stand der Einschlafhilfe 1 begünstigt.

Um die Lichtquelle 2 und/oder die Tonquelle 3 ansteuern zu können, ist die Steuereinrichtung 4 mit der Lichtquelle 2 und/oder der Tonquelle 3 signalverbunden oder signalverbindbar. Beispielsweise ist die Steuereinrichtung 4 mit der Lichtquelle 2 und/oder der Tonquelle 3 kabellos signalverbunden bzw. kabellos signalverbindbar, wie in der Figur 1 angedeutet ist. Zur kabellosen Übertragung kann die Einschlafhilfe 1 Bluetooth und/oder WLAN nutzen und dazu wenigstens eine (in den Figuren 1 und 2 nicht dargestellte) Einrichtung aufweisen.

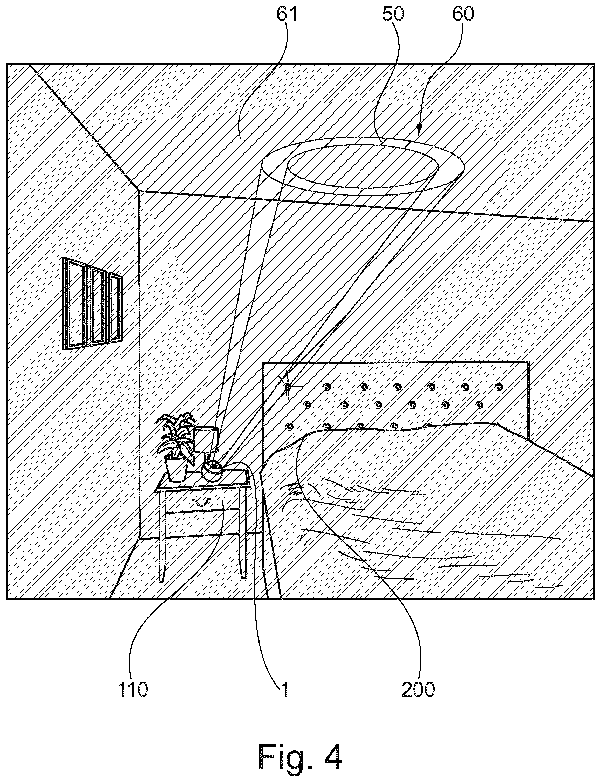

Die exemplarische Einschlafhilfe 1 umfasst beispielsweise eine Projektionseinrichtung 5, um das von der Lichtquelle 2 erzeugte Licht auf eine Fläche 60 zu projizieren, wie beispielhaft aus Figur 4 ersichtlich ist. Die Fläche 60 ist dort die Deckenfläche eines Raumes, wobei die exemplarische Einschlafhilfe 1 auf einem Nachtisch 110 neben einem Bett 200 aufgestellt ist. Die Beleuchtung der Fläche 60 ist besonders sichtbar, wenn der Raum abgedunkelt ist, sich also in einem für das Schlafen vorbereiteten Zustand befindet.

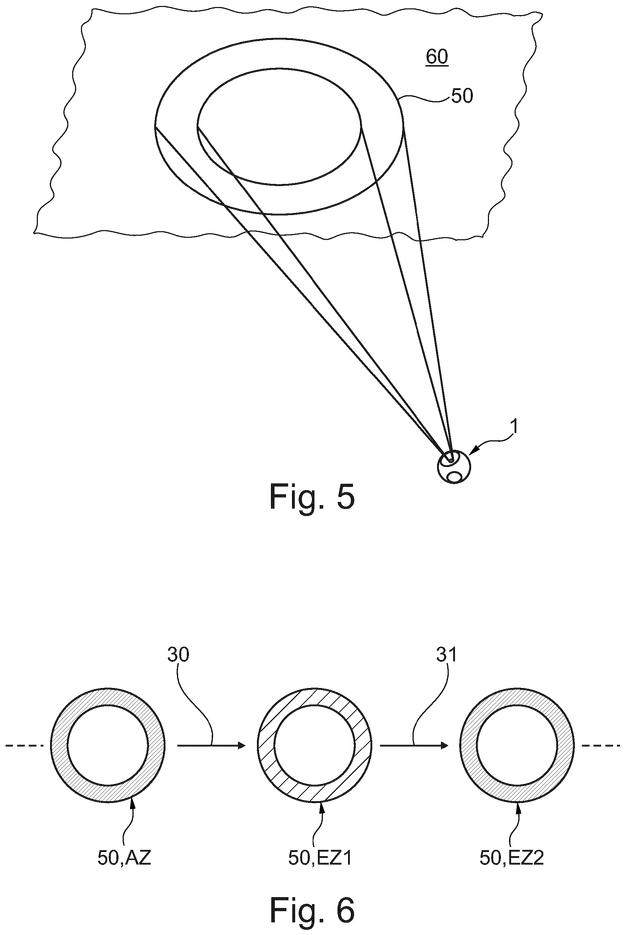

Beispielsweise ist die Projektionseinrichtung 5 eingerichtet, eine geometrische Figur 50 auf die Fläche 60 zu projizieren, wie aus der Figur 4 ersichtlich ist und zur besseren Veranschaulichung auch in Figur 5 dargestellt ist. Um die geometrische Figur 50 erzeugen zu können, weist die exemplarische Einschlafhilfe 1 beispielsweise eine Projektionsvorlage 6 auf, welche von der Lichtquelle 2 beleuchtet wird. Insofern dient in diesem Fall die Lichtquelle 2 als Beleuchtungsquelle zum Beleuchten der Projektionsvorlage 6.

Die Projektionsvorlage 6 kann eine Blendenöffnung 8 und ein darin vorzugsweise konzentrisch angeordnetes, lichtundurchlässiges Innenteil 9 umfassen oder daraus gebildet sein (Figur 2). Dadurch kann die projizierte geometrische Figur 50 in einer Ringkontur realisiert werden, wie beispielsweise aus den Figuren 4 und 5 ersichtlich ist. Bevorzugt weist dazu das Innenteil 9 einen Durchmesser D1 auf, welcher kleiner als der Durchmesser D2 der Blendenöffnung 8 ist. Beispielsweise sind die Blendenöffnung 8 und das Innenteil 9 rund, sodass die projizierte geometrische Figur 50 einen Rundring darstellt. Beispielsweise sind die Blendenöffnung 8 und das Innenteil 9 oval, insbesondere als zueinander gleichgerichtete Ovale ausgebildet, sodass die projizierte geometrische Figur 50 eine kreisrunde Ringform ist.

Wie insbesondere aus der Figur 2 ersichtlich ist, kann die Blendenöffnung 8 durch den Innenquerschnitt eines Hülsenelements 7 gebildet sein. Bevorzugt ist die Umfangswandung 7.1 des Hülsenelements 7 lichtundurchlässig ist, beispielsweise lichtabsorbierend oder lichtreflektierend, insbesondere um das von der Lichtquelle 2 erzeugte Licht möglichst vollständig durch das Hülsenelement 7 hindurch zu leiten. Beispielsweise befindet sich im Bereich eines axialen Endes des Hülsenelements 7 die Lichtquelle 2. Beispielsweise befindet sich im Bereich des gegenüberliegenden axialen Endes des Hülsenelements 7 das Innenteil 9. Beispielsweise ist das Innenteil 9 scheibenförmig, insbesondere als Rundscheibe, beispielsweise als Kreisscheibe ausgebildet.

Die exemplarische Einschlafhilfe 1 hat beispielsweise eine Hintergrundbeleuchtung 13. Durch die Hintergrundbeleuchtung 13 ist beispielsweise die Fläche 60 zu beleuchten, insbesondere farbig zu beleuchten, und zwar vorzugsweise um einen um die geometrische Figur 50 herum verlaufenden Flächenabschnitt 61. Die Hintergrundbeleuchtung 13 zielt darauf ab, eine entspannende Wirkung auf den Nutzer auszuüben, insbesondere wenn die Hintergrundbeleuchtung 13 als Ambientelicht realisiert ist.

Beispielweise umfasst die Hintergrundbeleuchtung 13 eine wenigstens weitere Lichtquelle 14 und wenigstens eine weitere Blendenöffnung 15. Beispielsweise sind die weitere Blendenöffnung 15 und die Blendenöffnung 8 konzentrisch zueinander angeordnet, insbesondere konzentrisch bezüglich einer gemeinsamen Achse A angeordnet. Beispielweise weist die weitere Blendenöffnung 15 einen größeren Durchmesser auf als die Blendenöffnung 8. Beispielsweise ist die Blendenöffnung 8 innerhalb der weiteren Blendenöffnung 15 angeordnet.

Beispielsweise ist die weitere Blendenöffnung 15 durch den Innenquerschnitt eines vorzugsweise länglichen Hülsenabschnitts 16 gebildet. Bevorzugt ist die Umfangswandung 16.1 des Hülsenabschnitts 16 lichtundurchlässig, insbesondere lichtabsorbierend oder lichtreflektierend. Beispielsweise befindet sich im Bereich eines axialen Endes des Hülsenabschnitts 16 die weitere Lichtquelle 14.

Beispielsweise ist die Lichtquelle 2 an einem Trägerteil 12 angeordnet. Beispielsweise ist an dem Trägerteil 12 auch das Hülsenelement 7 mit seinem einen Ende angeordnet, insbesondere aufgesetzt. Beispielsweise ist das Trägerteil 12 plattenförmig ausgebildet und erstreckt sich mit seiner Flächenseite quer, insbesondere orthogonal zur Achse A. Beispielsweise ist das Trägerteil 12 ein Kunststoffteil. Beispielsweise ist das Trägerteil 12 lichtundurchlässig. Beispielsweise ist an dem Trägerteil die wenigstens eine weitere Lichtquelle 14 angeordnet.

Die wenigstens eine weitere Lichtquelle 14 kann mehrere weitere, insbesondere vier Lichtquellen 14 umfassen. Beispielsweise sind die mehreren weiteren Lichtquellen 14 um die Lichtquelle 2 verteilt angeordnet. Beispielsweise ist jede der weiteren Lichtquellen 14 eingerichtet, ein Licht in unterschiedlichen Farben wahlweise zu erzeugen. Beispielsweise sind die mehreren weiteren Lichtquellen 14 so eingerichtet, dass wenigstens zwei oder mehrere der weiteren Lichtquellen 14 ein Licht in einer jeweils anderen Farbe erzeugt, wenn die Hintergrundbeleuchtung 13 eingeschaltet ist. Dies begünstigt die Nutzung der Hintergrundbeleuchtung 13 als Ambientebeleuchtung.

Beispielsweise ist das Innenteil 9 an einem Deckelteil 11 der Einschlafhilfe 1 angeordnet, insbesondere ausgebildet, beispielsweise angeformt oder ausgeformt. Beispielsweise ist das Innenteil 9 durch einen vertieften Abschnitt in dem Deckelteil 11 gebildet, wie in der Figur 2 angedeutet ist. Beispielsweise ist das Deckelteil 11 ein Kunststoffteil. Beispielsweise ist das Deckelteil 11 in seiner Abmessung so ausgebildet, dass es die weitere Blendenöffnung 15 abdeckt.

Beispielsweise ist das Deckelteil 11 im Bereich der weiteren Blendenöffnung 15 lichtdurchlässig. Beispielsweise ist das Deckelteil 11 gewölbt ausgebildet, beispielsweise nach innen in Richtung zum Inneren des Gerätegehäuses 10 gewölbt ausgebildet. Beispielsweise weist der Bereich des Deckelteils 11, welcher die weitere Blendenöffnung 15 überdeckt, eine Strukturierung auf. Dadurch kommt dem Deckelteil 11 zumindest in Bezug auf die Hintergrundbeleuchtung 13 eine Linsenfunktion, beispielsweise in Art einer Streulinse zu. Diesbezüglich kann die Strukturierung der weiteren Blendenöffnung 15 wabenförmig ausgebildet sein.

Beispielsweise bildet das Gerätegehäuse 10 mit seiner Außenfläche die Form einer Kugel aus. Beispielsweise ist die Aufstandsfläche 10.1 in einer Ebene angeordnet, welche das kugelförmige Gerätegehäuse 10 schneidet. Beispielsweise weist das Gerätegehäuse 10 eine Gehäuseöffnung 10.2 auf, welche beispielsweise von dem Deckelteil 11 überdeckt bzw. verschlossen ist. Beispielsweise liegt die Gehäuseöffnung 10.2 mit ihrer Öffnungsfläche in einer Ebene, welche das kugelförmige Gerätegehäuse 10 schneidet. Beispielsweise ist die Gehäuseöffnung 10.2 konzentrisch bzw. koaxial zur Achse A angeordnet. Beispielsweise ist die Gehäuseöffnung 10.2 gegenüberliegend zu der Aufstandsfläche 10.1 angeordnet.

Beispielsweise umfasst das Gerätegehäuse 10 mehrere, beispielsweise vier Gehäuseteile 10.3, 10.4, 10.5 und 10.6. Beispielweise bilden die Gehäuseteile 10.3, 10.4, 10.5 und 10.6 Kugelsegmente. Beispielsweise ist an dem, eine Kugelkalotte ausbildenden Gehäuseteil 10.3 die Aufstandsfläche 17.1 angeordnet, insbesondere ausgebildet. Beispielsweise ist an dem eine weitere Kugelkalotte ausbildenden Gehäuseteil 10.6 die Gehäuseöffnung 10.2 angeordnet, insbesondere ausgebildet. Beispielsweise weist eines der Gehäuseteile 10.3, 10.4, 10.5 und 10.6, insbesondere das Gehäuseteil 10.4, einen Flächenabschnitt 10.7 mit einer Vielzahl von Durchgängen auf, um darüber das von der Tonquelle 3 im Inneren des Gerätegehäuses 10 erzeugte akustische Signal nach außen zu übertragen.

Beispielsweise stützt sich das Trägerteil 12 an eine Haltestruktur 10.8 ab. Beispielweise ist das Trägerteil 12 an der Haltestruktur 10.8 befestigt. Beispielsweise ist die Haltestruktur 10.8 ein Bestandteil des Gerätegehäuses 10. Beispielsweise ist die Haltestruktur 10.8 an dem Gerätegehäuse 10 angeformt, insbesondere an einem der Gehäuseteile 10.3, 10.4, 10.5 und 10.6 angeformt. Beispielsweise ist die Haltestruktur 10.8 auch zur Befestigung der Tonquelle 3 genutzt.

Bevorzugt ist die exemplarische Einschlafhilfe 1 elektrisch betrieben. Bevorzugt ist zur elektrischen Energieversorgung der Lichtquelle 2 und/oder der Tonquelle 3 und/oder weiterer elektrische Verbraucher ein (in den Figuren 1 bis 3 nicht dargestelltes) Netzteil zum Anschließen an ein elektrisches Stromnetz vorgesehen. Alternativ kann auch ein vorzugsweise austauschbarer und vorzugsweise aufladbarer elektrischer Energiespeicher, wie beispielsweise ein Akkumulator, vorgesehen sein.

Bei der exemplarischen Einschlafhilfe 1 ist die Lichtquelle 2 beispielsweise als LED 17 ausgebildet oder umfasst wenigstens eine LED 17. Beispielsweise ist die LED eine Weißlicht-LED, welche also ein Licht mit einer Wellenlänge erzeugt, das als weißes Licht wahrgenommen wird. Bei der exemplarischen Einschlafhilfe 1 ist die wenigstens eine weitere Lichtquelle 14 beispielsweise als LED 18, insbesondere als RGB-LED, ausgebildet oder umfasst wenigstens eine solche LED 18. bei der exemplarischen Einschlafhilfe 1 ist die Tonquelle 3 beispielsweise ein Lautsprecher 19 oder umfasst wenigstens einen Lautsprecher 19.

Figur 6 zeigt beispielhaft eine mögliche geförderte Atemübung, welche durch die projizierte geometrische Figur 50 bewirkt werden kann und beispielsweise durch die Einschlafroutine vorgegeben werden kann. Ein Ausgangszustand AZ kann es beispielsweise sein, dass die geometrische Figur 50 in einem relativ dunklen Licht erscheint und hat beispielsweise eine vorgegebene erste Helligkeit. Davon ausgehend steuert nunmehr die Steuereinrichtung 4 die Lichtquelle 2 zum Ändern der Helligkeit des Lichts ins Hellere an, um damit ein Einatmen darzustellen. Daraufhin ändert sich die Helligkeit der geometrischen Figur 50 über eine vorgegebene Zeitdauer, welche beispielhaft anhand des Pfeils 30 angedeutet ist, ins Hellere und erreicht am Ende eine vorgegebene zweite Helligkeit, welche das Ende des Einatmens, also einen ersten Endzustand EZ1 darstellen soll.

Anschließend steuert die Steuereinrichtung 4 die Lichtquelle 2 zum Ändern der Helligkeit des Lichts ins dunklere an, um damit ein Ausatmen darzustellen. Daraufhin ändert sich die Helligkeit der geometrischen Figur 50 über eine vorgegebene Zeitdauer, welche beispielhaft anhand des Pfeils 31 angedeutet ist, ins Dunklere und erreicht am Ende eine vorgegebene dritte Helligkeit, welche das Ende des ausatmen, also einen zweiten Endzustand EZ2 darstellen soll. Die dritte Helligkeit im zweiten Endzustand EZ2 kann dabei der ersten Helligkeit im Ausgangszustand AZ entsprechen.

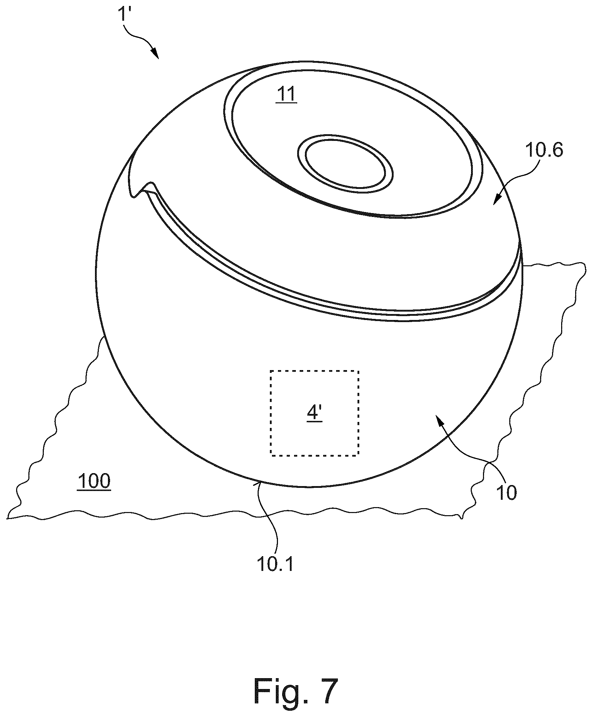

Figur 7 zeigt eine zweite exemplarische Ausführungsform einer elektrischen Einschlafhilfe 1' in einer perspektivischen Ansicht. Bauteile der elektrischen Einschlafhilfe 1', welche mit Bauteilen der elektrischen Einschlafhilfe 1 der Figuren 1 und 2 identisch oder funktionsgleich sind, sind mit gleichen Bezugszeichen versehen. Insofern wird auf die Beschreibung zu der elektrischen Einschlafhilfe 1 der Figuren 1 und 2 verwiesen.

Die exemplarische elektrische Einschlafhilfe 1' der Figur 7 unterscheidet sich von der exemplarischen Einschlafhilfe 1 der Figuren 1 und 2 beispielsweise dadurch, dass eine Steuereinrichtung 4' vorgesehen ist, welche dem Gerätegehäuse 10 zugeordnet ist, insbesondere in dem Gerätegehäuse 10 aufgenommen ist. Beispielsweise ist die Steuereinrichtung 4' mit der Lichtquelle 2 und/oder der Tonquelle 3 und/oder der weiteren Lichtquelle 14 kabelgebunden signalverbunden.

### Bezugszeichenliste

- 1, 1': Einschlafhilfe
- 2: Lichtquelle
- 3: Tonquelle
- 4, 4': Steuereinrichtung
- 5: Projektionseinrichtung
- 6: Projektionsvorlage
- 7: Hülsenelement
- 7.1: Umfangswandung
- 8: Blendenöffnung
- 9: Innenteil
- 10: Gerätegehäuse
- 10.1: Aufstandsfläche
- 10.2: Gehäuseöffnung
- 10.4: Gehäuseteil
- 10.5, 10.5, 10.6: Gehäuseteil
- 10.7: Flächenabschnitt
- 10.8: Haltestruktur
- 11: Deckelteil
- 12: Trägerteil
- 13: Hintergrundbeleuchtung
- 14: weitere Lichtquelle
- 15: Blendenöffnung
- 16: Hülsenabschnitt
- 17, 18: LED
- 19: Lautsprecher
- 20: Smartphone
- 30, 31: Pfeil
- 50: geometrische Figur
- 60: Fläche
- 100: Untergrund
- 110: Nachttisch
- 200: Bett

- A: Achse
- D1: Durchmesser (Blendenöffnung)
- D2: Durchmesser (Innenteil)

- AZ: Ausgangszustand
- EZ1: erster Endzustand
- EZ2: zweiter Endzustand

## Patentansprüche

1. Elektrische Einschlafhilfe (1; 1'), umfassend
eine Lichtquelle (2), welche eingerichtet ist, ein sichtbares Licht
zu erzeugen und die Helligkeit des Lichts zu ändern eine Tonquelle (3), welche eingerichtet ist, hörbare Töne und/oder hörbare Geräusche zu erzeugen,
eine Steuereinrichtung (4; 4'), welche eingerichtet ist, nach wenigstens einer vorzugsweise vorgegebenen Einschlafroutine sowohl die Lichtquelle (2) zum Ändern der Helligkeit des Lichts anzusteuern, um ein Einatmen oder Ausatmen einer geführten Atemmeditation visuell darzustellen, als auch die Tonquelle (3) zum Erzeugen wenigstens einer vorzugsweise gesprochenen und/oder musikalischen Tonfolge und/oder wenigstens eines Geräusches anzusteuern, um eine geführte Audiomeditation akustisch darzustellen.

2. Einschlafhilfe nach Anspruch 1, wobei die Steuereinrichtung (4; 4') eingerichtet ist, die Lichtquelle (2) zum Ändern der Helligkeit des Lichts ins Hellere anzusteuern, um eine Atemrichtung visuell darzustellen, und wobei die Steuereinrichtung (4; 4') eingerichtet ist, die Lichtquelle (2) zum Ändern der Helligkeit des Lichts ins Dunklere anzusteuern, um eine andere Atemrichtung visuell darzustellen.

3. Einschlafhilfe nach Anspruch 1 oder 2, wobei die Steuereinrichtung (4; 4') eingerichtet ist, die Lichtquelle (2) derart anzusteuern, dass die Helligkeit des Lichts über wenigstens eine vorgegebene Zeitdauer sich ändert, insbesondere heller oder dunkler wird, um einen Atemvorgang visuell darzustellen, und wobei die Steuereinrichtung (4; 4') eingerichtet ist, die Lichtquelle (2) derart anzusteuern, dass die Geschwindigkeit der Änderung der Helligkeit des Lichts über die wenigstens eine vorgegebene Zeitdauer sich ändert, insbesondere zunimmt oder abnimmt.

4. Einschlafhilfe nach einem der vorhergehenden Ansprüche, wobei die Steuereinrichtung (4; 4') eingerichtet ist, in Abhängigkeit wenigstens eines vorgegebenen Zeitpunktes die Lichtquelle (2) und/oder die Tonquelle (3) anzusteuern, um eine Information über ein bevorstehendes Einschlafen visuell und/oder akustisch auszugeben.

5. Einschlafhilfe nach einem der vorhergehenden Ansprüche, wobei die Einschlafroutine eine Datenbasis mit Informationen über Zeitpunkte zum Ansteuern der Lichtquelle (2) und Informationen über Zeitpunkte zum Ansteuern der Tonquelle (3) hat, und die Steuereinrichtung (4; 4') eingerichtet ist, danach die Lichtquelle (2) und die Tonquelle (3) anzusteuern.

6. Einschlafhilfe nach Anspruch 5, wobei die Zeitpunkte zum Ansteuern der Lichtquelle (2) und die Zeitpunkte zum Ansteuern der Tonquelle (3) aufeinander abgestimmt sind, um ein Einatmen oder Ausatmen visuell darzustellen, wenn eine zugehörige vorzugsweise gesprochene und/oder musikalische Tonfolge und/oder wenigstens ein zugehöriges Geräusch erfolgt ist oder erfolgt.

7. Einschlafhilfe nach einem der vorhergehenden Ansprüche, umfassend eine Projektionseinrichtung (5), welche eingerichtet ist, eine geometrische Figur (50) auf eine Fläche (60) zu projizieren, und dazu die Lichtquelle (2) als Beleuchtungsquelle zum Beleuchten einer Projektionsvorlage (6) nutzt.

8. Einschlafhilfe nach Anspruch 7, wobei die Projektionsvorlage (6) eine Blendenöffnung (8) und ein darin vorzugsweise konzentrisch angeordnetes Innenteil (9) umfasst, welches kleiner als die Blendenöffnung (8) ausgebildet und lichtundurchlässig ist, und wobei die Blendenöffnung (8) und das Innenteil (9) rund, insbesondere oval sind.

9. Einschlafhilfe nach Anspruch 7 oder 8, umfassend eine Hintergrundbeleuchtung (13) zum Beleuchten eines um die geometrische Figur (50) herum verlaufenden Flächenabschnitts (61).

10. Einschlafhilfe nach einem der vorhergehenden Ansprüche, umfassend ein Gerätegehäuse (10), in dem die Lichtquelle (2) und die Tonquelle (3) aufgenommen sind, wobei das Gerätegehäuse (10) eine Aufstandsfläche (10.1) zum Aufstellen auf einem Untergrund (100), insbesondere einem Nachttisch, hat.

11. Einschlafhilfe nach Anspruch 10, wobei die Steuereinrichtung (4') dem Gerätegehäuse (10) zugeordnet ist, insbesondere darin aufgenommen ist.

12. Einschlafhilfe nach einem der Ansprüche 1 bis 10, wobei die Steuereinrichtung (4) ein Bestandteil eines separaten Endgerätes, insbesondere eines Smartphones (20), Tablets oder Notebooks ist und eingerichtet ist, mit der Lichtquelle (2) und der Tonquelle (3) signalverbunden zu werden, insbesondere kabellos signalverbunden zu werden.

13. Einschlafhilfe nach einem der vorhergehenden Ansprüche, wobei die Lichtquelle (2) wenigstens eine LED (17) und die Tonquelle (3) wenigstens einen Lautsprecher (19) umfassen.

14. Verfahren zur Steuerung einer Einschlafhilfe (1; 1') nach einem der vorhergehenden Ansprüche, bei dem mittels der Steuereinrichtung (4; 4') nach einer vorgegebenen Einschlafroutine sowohl die Lichtquelle (2) zum Ändern der Helligkeit des Lichts angesteuert wird, um ein Einatmen oder Ausatmen einer geführten Atemmeditation visuell darzustellen, als auch die Tonquelle (3) zum Erzeugen einer vorzugsweise gesprochenen und/oder musikalischen Tonfolge und/oder wenigstens eines Geräusches angesteuert wird, um eine geführte Audiomeditation akustisch darzustellen.

15. Computerprogrammprodukt mit einem Programmcode, der einen Computer zur Durchführung der Schritte des Verfahrens nach Anspruch 14 anweist, wenn der Programmcode auf dem Computer ausgeführt wird.

16. Computer, insbesondere Smartphone (20), mit einer Steuereinrichtung (4; 4') für eine Einschlafhilfe (1; 1') nach einem der Ansprüche 1 bis 13, umfassend ein Computerprogrammprodukt nach Anspruch 15.
